# EUROPEAN PATENT SPECIFICATION

(11) **EP 4 429 748 B1**
(45) Date of publication and mention of the grant of the patent: **07.10.2026**
(21) Application number: 22829983.0
(22) Date of filing: 08.11.2022
(51) Int. Cl.: A61M 35/00

(54) **SKIN DISINFECTING SCRUBBING DEVICE WITH ACCURATE DISINFECTANT SOLUTION VOLUME DISPENSING CONTROL**
HAUTDESINFIZIERENDES SCHRUBGERÄT MIT PRÄZISER VOLUMENDOSIERUNG DER DESINFEKTIONSLÖSUNG
DISPOSITIF DE NETTOYAGE DÉSINFECTANT DE LA PEAU AVEC CONTRÔLE PRÉCIS DU VOLUME DE LA SOLUTION DÉSINFECTANTE

(30) Priority: 09.11.2021 US 202163277340 P
(43) Date of publication of application: 18.09.2024
(73) Proprietor: Becton, Dickinson and Company, Franklin Lakes, NJ 07417-1880 (US)
(72) Inventor: RYAN, Kevin M., Whitehouse Station, New Jersey 08889 (US); PRASAD, Shishir, Ramsey, New Jersey 07446 (US); KULKARNI, Aniket Anand, Bangalore 560 097 Karnataka (IN); RODRIGUES, Ruben, Bangalore 560 043 Karnataka (IN); NALAWADE, Praveen, Belgaum 590 016 Karnataka (IN); MALVIYA, Rahul, Bangalore 560 043 Karnataka (IN); JADHAV, Amarsinh Deeliprao, Bangalore 560 077 Karnataka (IN)
(74) Representative: dompatent
(86) International application number: PCT/US2022/049214
(87) International publication number: WO 2023/086317

(56) References cited:
- EP-A1- 1 796 777
- KR-B1- 101 505 501
- US-A1- 2003 075 200
- US-A1- 2004 179 888
- US-A1- 2021 186 182

## Description

### TECHNICAL FIELD

The present disclosure relates to fluid applicators, and in particular the present disclosure relates to a fluid applicator with precise volume dispensing control for dispensing fluids such as a disinfectant in a controlled manner.

### BACKGROUND

Fluid applicators are commonly used in the art to dispense disinfectant solution onto a patient's skin prior to or after medical procedures such as surgery or even when inserting an intravenous catheter or other vascular access device. Such devices are effective at locally disinfecting and reducing microbial infection of the surgical site ultimately to reduce the risk of bloodstream infections following the procedure.

Fluid applicators within the art lack precise control of the release of disinfectant. The clinician performing the procedure has to estimate the volume of disinfecting solution necessary to adequately cover the surface area for a given procedure. In doing this estimation, clinicians will often choose to dispense an excess amount of fluid to ensure full wet-out of the skin as opposed to rounding-down to a smaller volume, which could lead to not completely wetting out the area and hence not fully disinfected areas.

An excess amount of fluid dispensed can lead to pooling & dripping from the target disinfecting area. These drips can run onto sterile field drapes, down the sides of the patient, and onto the clinician's gloves and clothing. Dripping, in conjunction with the fact that the disinfecting solution is often sticky (such as for Chlorahexidine), can cause parts of the clinician's gloves, foreign matter or bacterial contaminates sticking to the sterile draping areas where the solution dripped onto. In addition, pooling and dripping of the fluid takes significantly more time to dry, and allowing time for the fluid to dry is an extremely important requirement. The fluid is typically a disinfectant solution often containing 70% IPA, which is flammable if pooled. The pooled fluid can ignite by static electricity discharge from draping materials or by tools which are used for medical procedures such as laser surgery tools.

Some of the most common type of fluid applicators are dispensing devices having a breakable ampoule or cartridge containing disinfectant. The dispensing device can have a chamber for receiving the ampoule, an applicator head and a means for breaking the ampoule, thusly ejecting disinfectant onto the applicator head. To select a greater volume of disinfectant for greater surfaces areas which require disinfection, a larger ampoule can be selected. In operation, the clinician will break the ampoule resulting in rapid and uncontrolled gushing of the fluid from the ampoule. The fluid will first dispense at a higher rate and will dispense at a lesser rate as the ampoule empties.

Clinicians will often attempt to adjust for the variable rate by scrubbing faster (essentially covering more area faster) than otherwise recommended and then stopping scrubbing before the required minimum scrub time for a given disinfection procedure or required by the stated device instructions for the particular ampoule and applicator. Because the scrubbing and disinfecting is stopped before the minimum required time, or because there is no fluid remaining for the minimum required time, the skin area may not be fully or adequately disinfected, leading to potential increased risk of infection.

Due to the rapid and uncontrolled gushing of fluid upon breakage of an ampoule, common applicators have implemented various configurations to limit or control the flow of fluid. To overcome the challenges faced by clinicians, fluid applicators can be provided with additional ampoules of varying volume sizes available for the clinician to choose from. However, this solution results in fluid applicator kits with additional or excess ampoules, many of which will be discarded. Furthermore, it can be a challenge for the clinician to estimate which exact volume is needed due to unknown factors such as skin abortion of the disinfection solution and calculation of skin surface area due to complicated skin surface geometry, and time constraints of the given procedure. Further configurations can include means of limiting the flow of fluid downstream from the ampoule. Such means can include tortious paths or absorbent materials which allow the gushing fluid to slowly permeate through the tortious paths or absorbent materials before exiting the common applicator. This results in the clinician having to wait for the initial fluid to pass through the tortious paths or absorbent materials in order to wet-out the bottom of the common applicator, resulting in a time delay between breakage of the ampoule and application of the disinfectant fluid.

Thus, there is a need to provide a fluid applicator which can provide a controlled and constant flow rate of fluid onto a scrubbing sponge of the applicator.
US 2021/0186182 A1 discloses a back solution applicator.
KR 101505501 B1 discloses an applicator for dispensing medical liquefied adhesive.
US 2003/0075200 A1 discloses a device for applying a product.

### SUMMARY

A first aspect of the present disclosure relates to a fluid applicator having a housing having an open proximal end, a distal base and a cavity configured to receive a pumping device, a pumping device at least partially disposed within the open proximal end of the housing and fluid disposed between the barrel and the plunger rod, The pumping device has a barrel and a plunger at least partially disposed within the barrel. The barrel has a distal end and an elongate tip extending from the distal end of the barrel. The elongate tip is positioned a distance from the distal base of the housing. The distal base having a fluid applicator sponge extending distally from the distal base.

In some embodiments, the fluid applicator further includes a flow distribution sponge disposed adjacent and proximal to the distal base.

In some embodiments, the flow distribution sponge is configured to uniformly distribute fluid from the elongate tip of the pumping device.

According to the invention, the fluid applicator further includes a frangible seal disposed over the elongate tip configured to break upon advancement of the plunger rod into the barrel of the pumping device.

In some embodiments, the fluid applicator further includes a friction tip cap disposed over the elongate tip configured to eject upon advancement of the plunger rod into the barrel of the pumping device.

In some embodiments, the fluid applicator further includes barrel includes a flange surrounding the open proximal end of the barrel, the flange configured as a hard stop for limiting insertion of the pumping device into the housing.

In some embodiments, the one or more longitudinal ribs extend from an inside surface of the housing are configured to create an interference fit with the barrel.

In some embodiments, the one or more longitudinal ribs may be tabs or an inner flange.

In some embodiments, the one or more longitudinal ribs are positioned a distance within the housing such that the distal end of the barrel of the pumping device abuts the one or more longitudinal ribs.

In some embodiments, the distal base is at an angle relative to the housing. In some embodiments, the distal base is perpendicular to the housing.

In some embodiments, the hollow barrel of the pumping device is made from a high barrier injection molded resin or glass configured to resist EtO Gas.

In some embodiments, the pumping device is resistant EtO Gas.

A second aspect of the present disclosure is directed to a method of assembling and sterilizing the fluid applicator of the present disclosure. The method comprises the steps of:
pre-filling the pumping device with disinfectant, sealing the elongate tip of the pumping device with a frangible seal, sterilizing the pumping device with EtO sterilization, advancing the hollow barrel of the pumping device into the cavity of the housing, and, packaging and sterilizing the fluid applicator with a non-hazardous sterilization.

### BRIEF DESCRIPTION OF THE DRAWINGS

FIG. 1 illustrates an exploded view of a fluid applicator in accordance with one or more embodiments of the present disclosure;
FIG. 2A illustrates a side view of a fluid applicator in accordance with one or more embodiments of the present disclosure;
FIG. 2B illustrates a side view of a fluid applicator in accordance with one or more embodiments of the present disclosure;
FIG. 3A illustrates a detailed cross-sectional view of a fluid applicator in accordance with one or more embodiments of the present invention;
FIG. 3B illustrates a detailed cross-sectional view of a fluid applicator in accordance with one or more embodiments of the present disclosure without the flow distribution sponge;
FIG. 4 illustrates a perspective view of a pump of a fluid applicator in accordance with one or more embodiments of the present disclosure;
FIG. 5 illustrates a side view of a pump of a fluid applicator in accordance with one or more embodiments of the present disclosure; and,
FIG. 6 illustrates a cross-sectional view of a fluid applicator in accordance with one or more embodiments of the present disclosure.

### DETAILED DESCRIPTION

Before describing several exemplary embodiments of the disclosure, it is to be understood that the disclosure is not limited to the details of construction or process steps set forth in the following description. The disclosure is capable of other embodiments and of being practiced or being carried out in various ways.

For purposes of the description hereinafter, the terms "proximal", "distal", "longitudinal", and derivatives thereof shall relate to the disclosure as it is oriented in the drawing figures. However, it is to be understood that the disclosure may assume alternative variations and step sequences, except where expressly specified to the contrary. It is also to be understood that the specific devices and processes illustrated in the attached drawings, and described in the following specification, are simply exemplary embodiments of the disclosure. Hence, specific dimensions and other physical characteristics related to the embodiments disclosed herein are not to be considered as limiting.

As used herein, the use of "a," "an," and "the" includes the singular and plural.

As would be readily appreciated by skilled artisans in the relevant art, while descriptive terms such as "thread", "taper", "tab", "wall", "proximal", "side", "distal" and others are used throughout this specification to facilitate understanding, it is not intended to limit any components that can be used in combinations or individually to implement various aspects of the embodiments of the present disclosure.

As used herein, the terms "package" or "packaging" includes any material used to wrap or protect a medical device or product, such as plastic ampoules, drug vials with rubber stoppers, IV solution bags, IV solution pouches and syringes. Packaging can be rigid or flexible. Packaging includes, but is not limited to, medical packaging, pharmaceutical packaging, and child-resistant packaging. Medical and pharmaceutical packaging can include plastic trays with webbing, blister packs, flow wrap and 3 or 4 sided pouches.

As used herein, the term "microorganism" refers to a microbe or organism that is unicellular or lives in a colony of cellular organisms. Microorganisms are very diverse; they include, but are not limited to bacteria, fungi, archaea, and protozoans.

As used herein, the term "sterilization" refers to a wide variety of techniques employed to attenuate, kill or eliminate harmful or infectious agents. Examples of sterilization procedures include, for example, steam sterilization, ethylene oxide sterilization (EtO sterilization), gas plasma sterilization, ozone sterilization, hydrogen peroxide sterilization, heat sterilization, nitrous dioxide sterilization, or a combination thereof. During manufacturing, pre-filled medical devices (such as drugs or other injectable/infusible solutions which are packaged in gas-permeable containers such as plastic ampoules, drug vials with rubber stoppers, IV solution bags, IV solution pouches and pre-filled flush syringes) are sterilized with non-toxic or non-hazardous sterilization processes such as a steam sterilization in an autoclave. Other medical devices which are less sensitive can undergo subsequent EtO sterilization without any adverse effects to the device sterilized. EtO sterilization can be considered more hazardous to pre-filled medical devices.

As used herein, the term "ethylene oxide (EtO) sterilization" is a common in methods used to prepare a kit of multiple medical devices or accessories for use in a sterile field. Currently, pre-filled saline syringes, plastic ampoules, drug vials with rubber stoppers, IV solution bags, IV solution pouches are packaged in gas permeable packaging which is also permeable to EtO gas. Such devices can be made of glass or high barrier injection molded resin capable of protecting the device itself or the contents within from EtO gas. However, exposure of a plastic ampoules, drug vials with rubber stoppers, IV solution bags, IV solution pouches or pre-filled syringe to EtO gas results in an undesirable effect of increasing the pH of the contents of the plastic ampoules, drug vials with rubber stoppers, IV solution bags, IV solution pouches or pre-filled syringe, e.g. saline. To overcome this undesired effect, medical devices which are resistive to EtO gas are sterilized separately from medical devices which are not. Often, these separate medical devices are later combined in a kit which can then be sterilized as a whole.

The term "high barrier" material as used herein refers to a material which is resistive to EtO gas and can be sterilized with EtO gas (essentially undergoing an entire EtO sterilization). Such materials can include, but are not limited to, glass, high barrier resins, top web films or aluminum alloy films.

Embodiments of the present disclosure are directed to a fluid applicator with precise volume dispensing control for dispensing fluids such as a disinfectant in a controlled manner. The fluid applicator of the embodiments described utilizes a pumping device attachable to a housing having an applicator sponge. The pumping device ensures a controlled flow of fluid to the applicator sponge. In further embodiments, a flow distribution sponge is disposed within the housing and is configured to uniformly distribute fluid from the pumping device. The fluid applicator is capable of undergoing EtO sterilization.

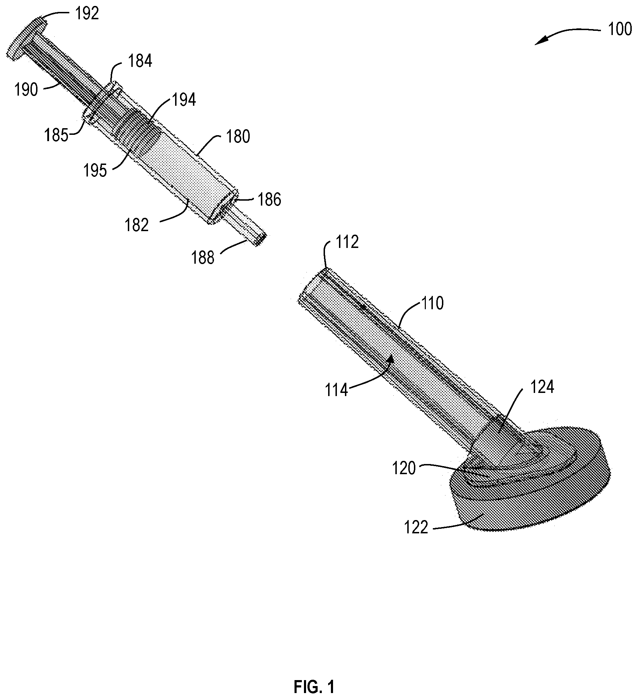

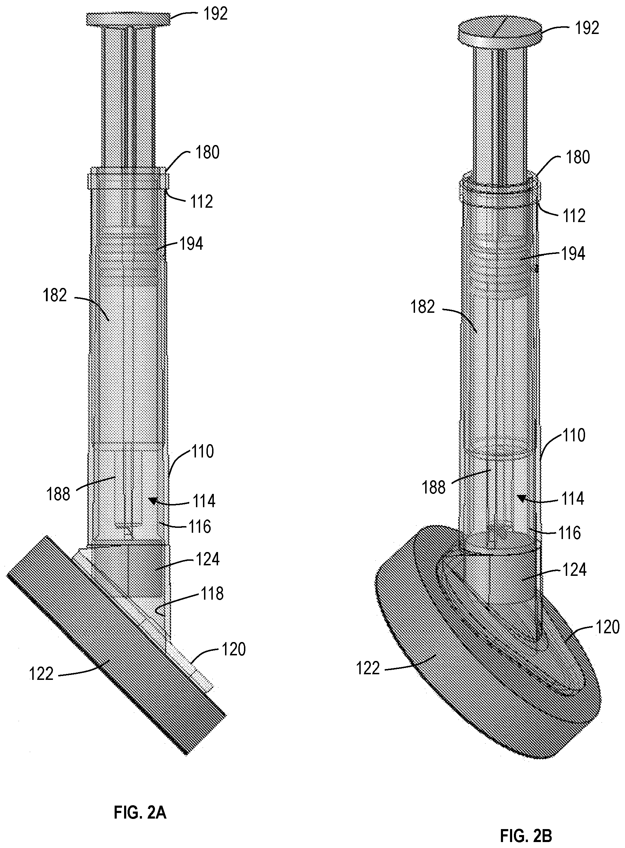

FIG. 1 illustrates an exploded view of a fluid applicator 100 in accordance with one or more embodiments. FIGS. 2A and 2B illustrate assembled side views of the fluid applicator 100. As shown in FIGS. 1, 2A and 2B, the fluid applicator comprises a housing 110 and a pumping device 180. The pumping device 180 is at least partially inserted into the housing 110 and can provide a controlled and constant flow of fluid. In some embodiments, the pumping device 180 is a syringe.

The pumping device 180 comprises a hollow barrel 182 having an open proximal end 184 and a distal end 186. In some embodiments, the distal end 186 comprises an opening or a nozzle configured to eject fluid from the barrel 182. In some embodiments, the opening or nozzle is an elongate tip 188 extending from the distal end 186 in a distal direction configured to direct fluid out of the barrel 182. In some embodiments, the open proximal end 184 comprises a flange 185 extending around the open proximal end 184. The flange 185 is configured to provide a surface onto which distal pressure can be applied to at least partially advance the barrel 182 into the housing 110. As explained in further detail below, the flange 185 can be configured as a hard stop, limiting the advancement of the barrel 182 into the housing 110.

The pumping device 180 further comprises a plunger 190 movably positioned within the hollow barrel 182. The plunger 190 has a proximal end 192 and a distal end 194, the distal end 194 creating a fluid seal with an inner surface of the hollow barrel 182. Withdrawing the plunger 190 relative to the barrel 182 creates a suction force at the elongate tip 188 and advancing the plunger 190 relative to the barrel 182 creates pressure buildup at the elongate tip 188. In some embodiments, the barrel 182 is filled with a fluid such as a disinfectant by withdrawing the plunger 190 relative to the barrel 182. In some embodiments, the pumping device 180 is pre-filled with fluid prior to packaging. In some embodiments, the pumping device 180 is filled with fluid during a disinfectant procedure, as explained in further detail below. In some embodiments, the pumping device 180 is a conventional syringe.

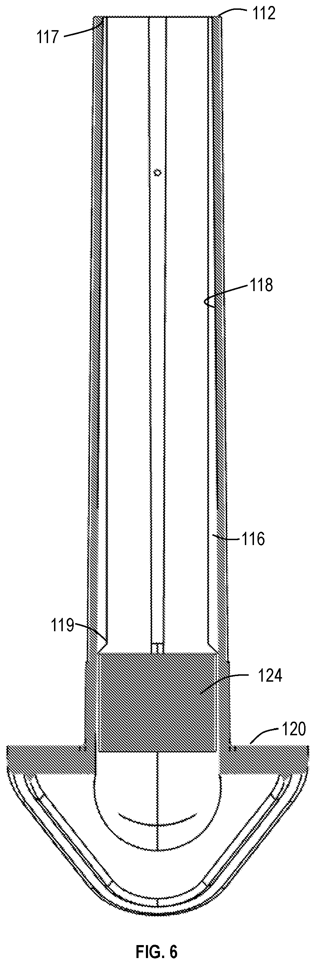

The housing 110 comprises a hollow elongate body having an open proximal end 112 and a distal base 120. As best shown in FIGS. 2A and 2B, the barrel 182 of the pumping device 180 is inserted into a cavity 114 the housing 110. In some embodiments, the flange 185 of the pumping device 180 is configured as a hard stop to limit the insertion distance of the barrel 182 into the housing 110. In particular, the barrel 182 is inserted into the housing 110 until the flange 185 abuts the open proximal end 112 of the housing 110. As best shown in FIG. 2A and FIG. 6, in some embodiments, one or more longitudinal ribs 116 extending from an inside surface 118 are configured to create an interference fit with the barrel 182 or limit the insertion distance of the barrel 182 into the housing 110. In some embodiments, the one or more longitudinal ribs 116 are tabs. In some embodiments, the one or more longitudinal ribs 116 are an inner flange. As shown in FIG. 6, the one or more longitudinal ribs 116 extend from the open proximal end 112 to a distance towards the distal base 120. The one or more longitudinal ribs 116 having a proximal end 117 and a distal end 119. The one or more longitudinal ribs 116 slope inwardly into the cavity 114 of the housing 110 such that the distal end 119 extends further into the cavity 114 than the proximal end 117. The slope of the one or more longitudinal ribs 116 is configured to create an interference fit with the hollow barrel 182 of the pumping device 180. Due to the slope, the one or more longitudinal ribs 116 is capable of accommodating syringes or pumping devices having slightly varying barrel outer diameters. As also shown in FIG. 6, the distal end 119 of the one or more longitudinal ribs 116 prevents the flow distribution sponge 124 from advancing proximally past the distal end 119 due to the flow distribution sponge 124 being wider than the distal end 119 of the one or more longitudinal ribs 116.

The distal base 120 has a fluid applicator sponge 122 distally affixed to the distal base 120. In some embodiments, the fluid applicator sponge 122 is removably affixed to the distal base 120. In some embodiments, the fluid applicator sponge 122 is removably affixed to the distal base 120 by a non-permanent medical grade adhesive or hook-and-loop. In some embodiments, the fluid applicator sponge 122 is non removably affixed to the distal base 120 by a permanent medical grade adhesive. At least a portion of the fluid applicator sponge 122 is in fluid communication with the cavity 114 of the housing such that fluid flowing from the pumping device 180 into the cavity 114 will permeate though and across the fluid applicator sponge 122. In some embodiments, the distal base 120 is perpendicular to the housing 110. In some embodiments, the distal base 120 is at an angle relative to the housing 110.The distal base 120 can be angled to ergonomically assist in the application of fluid by the fluid applicator sponge 122.

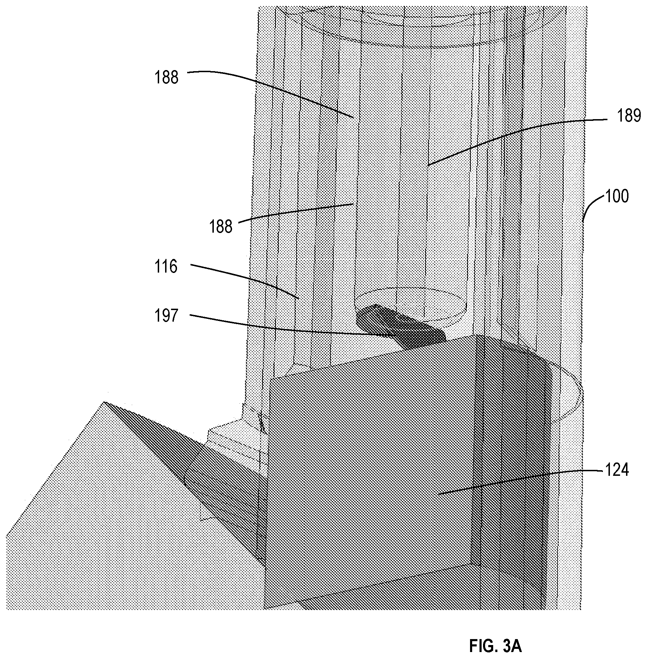

In some embodiments, as best shown in FIG. 3A, a flow distribution sponge 124 is positioned within the cavity 114 and is disposed adjacent and proximal to the distal base 120. The flow distribution sponge 124 is configured to uniformly distribute fluid from the elongate tip 188 of the pumping device 180 to the fluid applicator sponge 122. Due to the pumping device 180 providing a steady flow of fluid as explained in further detail below, the fluid applicator 100 of the present disclosure does not require the flow distribution sponge 124 to dampen or limit rapid and uncontrolled gushing of fluid upon breakage of an ampoule. Thus, an advantage of the present disclosure over prior art is that the pumping device 180 can be adjacent to the fluid applicator sponge 122 without the need of the flow distribution sponge 124, or more generally a tortious paths or absorbent materials for the purposes of limiting or slowing of gushing or rapid flow. In particular, common applicators having a breakable ampoule or more generally a single-release or uncontrolled release device implement flow limiting materials before the applicator sponge to dampen the rapid flow.

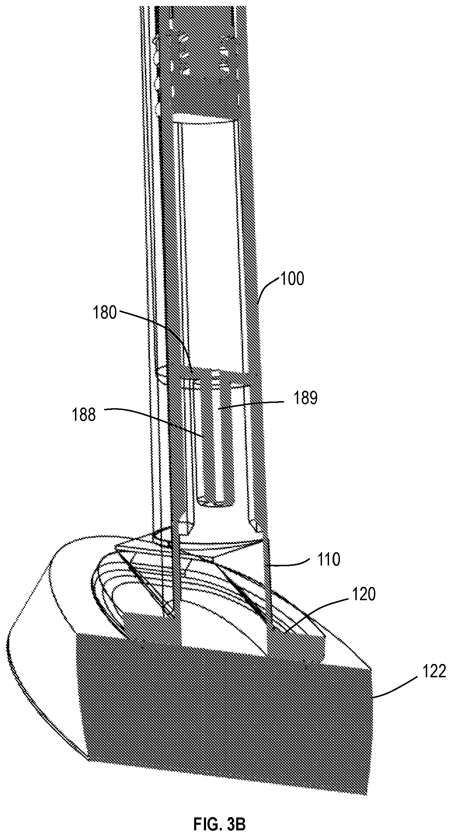

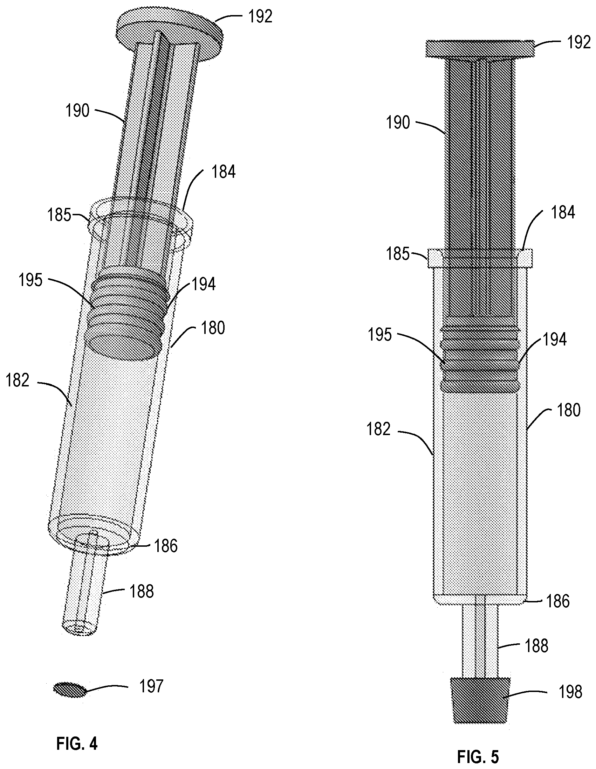

As shown in FIGS. 3 through 6, the pumping device 180 further comprises a barrier configured to seal a lumen 189 of the elongate tip 188 during packaging and prior to use of the fluid applicator 100, the lumen 189 being in fluid communication with the hollow barrel 182. According to the invention, as shown in FIGS. 3 and 4, the barrier is a frangible seal 197 or film. In some embodiments, as shown in FIG. 5, the barrier is a friction tip cap 198. Both the frangible seal 197 and friction tip cap 198 are configured to break or release from the elongate tip 188 upon sufficient application of hydraulic force due to advancement of the plunger 190 distally into the hollow barrel 182. In some embodiments, as shown in 3A, the pumping device 180 is provided with the flow distribution sponge 124. In some embodiments, as shown in 3B, the pumping device 180 is advantageously provided without the flow distribution sponge 124.

In particular, the frangible seal 197 is removably affixed to the elongate tip 188 and upon sufficient application of hydraulic force the frangible seal 197 breaks or at least partially breaks apart from the elongate tip 188, thereby allowing fluid to flow from the hollow barrel 182 to the cavity 114 of the housing, through the flow distribution sponge 124 and finally to the fluid applicator sponge 122. Similarly, the friction tip cap 198 removably surrounds the elongate tip 188 forming a friction or interference fit. Upon sufficient application of hydraulic force the friction or interference fit breaks and the friction tip cap 198 is distally ejected from the elongate tip 188, thereby allowing fluid to flow from the hollow barrel 182 to the cavity 114 of the housing, through the flow distribution sponge 124 and finally to the fluid applicator sponge 122. In either embodiment, as shown in FIG. 3, the flange 185 of the open proximal end 184 of the hollow barrel 182, the housing 110 itself and the one or more longitudinal ribs 116 are configured to position the elongate tip 188 a distance away from the flow distribution sponge 124 such that breaking of the frangible seal 197 or ejection of the friction tip cap 198 will not hinder fluid flow by providing adequate distance to break or eject.

In embodiments without the flow distribution sponge 124, upon sufficient application of hydraulic force the frangible seal 197 (or friction top cap 198) ejects, breaks or at least partially breaks apart from the elongate tip 188, thereby allowing fluid to flow from the hollow barrel 182 to the cavity 114 of the housing, directly to the fluid applicator sponge 122.

In some embodiments, components of the pumping device 180 are made from materials which are resistant to EtO gas and thus able to undergo EtO sterilization. In particular, in some embodiments, the hollow barrel 182 is made of glass or high barrier injection molded resin. Likewise, the plunger 190 can be made from metal or high barrier injection molded resin and a stopper 195 disposed on the distal end 194 of the plunger 190 can be made of high barrier injection molded resin or rubber. Finally, the frangible seal 197 can be made from heat sealable high barrier top web film and the friction tip cap 198 can be made from high barrier injection molded resin or rubber.

Further embodiments are directed to a method of assembling and sterilizing a fluid applicator 100 comprising the steps of pre-filling the pumping device 180 with disinfectant, sealing the elongate tip 188 of the pumping device 180 with the frangible seal 197 or the friction tip cap 198, sterilizing the pumping device 180 with EtO sterilization, advancing the hollow barrel 182 of the pumping device 180 into the cavity 114 of the housing 110 until one or more of the aforementioned hard stops are hit, and, packaging and sterilizing the fluid applicator 100 with a non-hazardous sterilization.

Due to components of the pumping device 180 are made from materials which are resistant to EtO gas and thus able to undergo EtO sterilization, the pumping device 180 and the disinfectant pre-filled within the pumping device 180 are not affected by the harsh side effects of EtO sterilization. In particular, chemical stability of the fluid or disinfectant is not altered by EtO sterilization.

In operation, the clinician pressing on the plunger 190 of the pumping device 180 with sufficient force to cause an increased internal pressure in the hollow barrel 182. Once the high enough to burst or peel the frangible seal 197 or the friction tip cap 198 from the elongate tip 188 of the pumping device. In some embodiments, disinfectant can then flow out from the pumping device 180 in a controlled and constant matter into the flow distribution sponge 124 and subsequently the fluid applicator sponge 122. In embodiments without the flow distribution sponge 124, disinfectant flows out from the pumping device 180 in a controlled and constant matter directly into the fluid applicator sponge 122 The clinician can control the volume and flow rate of the fluid dispensed by further pressing the plunger 190 into the hollow barrel 182. Hence, the amount of fluid can be precisely controlled directly by the clinician, as to avoid excessive dripping or pooling regardless of desired coverage rate, skin absorption rates and scrubbing force. In some embodiments, the syringe also includes metering marks that can be configured to gauge how much fluid has been displaced. was actually used on the area.

While the present disclosure has been shown and described with reference to certain exemplary embodiments thereof, it will be understood by those skilled in the art that various changes in form and details may be made therein without departing from the scope of the embodiments of the present invention, as defined by the appended claims. Furthermore, any of the features or elements of any exemplary implementations of the embodiments of the present disclosure as described above and illustrated in the drawing figures can be implemented individually or in any combination(s) as would be readily appreciated by skilled artisans without departing from the scope of the embodiments of the present invention, as defined by the appended claims.

In addition, the included drawing figures further describe non-limiting examples of implementations of certain exemplary embodiments of the present disclosure and aid in the description of technology associated therewith. Any specific or relative dimensions or measurements provided in the drawings other as noted above are exemplary and not intended to limit the scope or content of the inventive design or methodology as understood by artisans skilled in the relevant field of invention.

Reference throughout this specification to "one embodiment," "certain embodiments," "one or more embodiments" or "an embodiment" means that a particular feature, structure, material, or characteristic described in connection with the embodiment is included in at least one embodiment of the disclosure. Thus, the appearances of the phrases such as "in one or more embodiments," "in certain embodiments," "in one embodiment" or "in an embodiment" in various places throughout this specification are not necessarily referring to the same embodiment of the disclosure.

Although the disclosure herein has provided a description with reference to particular embodiments, it is to be understood that these embodiments are merely illustrative of the principles and applications of the present disclosure. It will be apparent to those skilled in the art that various modifications and variations can be made to the method and apparatus of the present disclosure without departing from the scope of the invention. Thus, it is intended that the present disclosure include modifications and variations that are within the scope of the appended claims.

## Claims

1. A fluid applicator (100) comprising:
a housing (110) having an open proximal end, a distal base and a cavity (114) configured to receive a pumping device (180), the distal base having a fluid applicator sponge (122) extending distally from the distal base;
the pumping device (180) at least partially disposed within the open proximal end of the housing (110), the pumping device (180) having a barrel (182) and a plunger (190) at least partially disposed within the barrel, the barrel (182) having a distal end and an elongate tip (188) extending from the distal end of the barrel, the elongate tip positioned a distance from the distal base of the housing (110); and,
fluid disposed between the barrel (182) and the plunger (190), and
**characterized in that**
a frangible seal (197) is disposed over the elongate tip (188) configured to break upon advancement of the plunger (190) into the barrel (182) of the pumping device (180).

2. The fluid applicator (100) of claim 1 further comprising a flow distribution sponge (124) disposed adjacent and proximal to the distal base.

3. The fluid applicator (100) of claim 2, wherein the flow distribution sponge (124) is configured to uniformly distribute fluid from the elongate tip of the pumping device (180).

4. The fluid applicator (100) of claim 1 further comprising a friction tip cap (198) disposed over the elongate tip configured to eject upon advancement of the plunger (190) into the barrel (182) of the pumping device (180).

5. The fluid applicator (100) of claim 1, wherein the barrel (182) further comprises a flange (185) surrounding the open proximal end of the barrel, the flange (185) configured as a hard stop for limiting insertion of the pumping device (180) into the housing (110).

6. The fluid applicator (100) of claim 1, wherein one or more longitudinal ribs (116) extend from an inside surface of the housing (110) being configured to create an interference fit with the barrel.

7. The fluid applicator (100) of claim 6, wherein the one or more longitudinal ribs (116) are tabs.

8. The fluid applicator (100) of claim 6, wherein the one or more longitudinal ribs (116) are positioned a distance within the housing (110) such that the distal end of the barrel (182) of the pumping device (180) abuts the one or more longitudinal ribs (116).

9. The fluid applicator (100) of claim 1, wherein the distal base is at an angle relative to the housing (110).

10. The fluid applicator (100) of claim 1, wherein the distal base is perpendicular to the housing (110).

11. The fluid applicator (100) of claim 1, wherein the hollow barrel (182) of the pumping device (180) is made from a high barrier injection molded resin or glass configured to resist Ethylene Oxide Gas.

12. The fluid applicator (100) of claim 1, wherein the pumping device (180) is resistant Ethylene Oxide Gas.

13. A method of assembling and sterilizing the fluid applicator (100) of claim 1 comprising the steps of:
pre-filling the pumping device (180) with disinfectant,
sealing the elongate tip of the pumping device (180) with a frangible seal (197), sterilizing the pumping device (180) with Ethylene Oxide Gas (EtO) sterilization,
advancing the hollow barrel (182) of the pumping device (180) into the cavity (114) of the housing (110), and,
packaging and sterilizing the fluid applicator (100) with a non-hazardous sterilization.

## Patentansprüche

1. Fluidapplikator (100), der aufweist:
ein Gehäuse (110) mit einem offenen proximalen Ende, einer distalen Basis und einem Hohlraum (114), der zur Aufnahme einer Pumpvorrichtung (180) ausgebildet ist, wobei die distale Basis einen Fluidapplikatorschwamm (122) aufweist, der sich von der distalen Basis in distaler Richtung erstreckt;
wobei die Pumpvorrichtung (180) zumindest teilweise innerhalb des offenen proximalen Endes des Gehäuses (110) angeordnet ist, wobei die Pumpvorrichtung (180) einen Zylinder (182) und einen Kolben (190) aufweist, der zumindest teilweise in dem Zylinder angeordnet ist, wobei der Zylinder (182) ein distales Ende und eine längliche Spitze (188) aufweist, die sich vom distalen Ende des Zylinders erstreckt, wobei die längliche Spitze in einem Abstand von der distalen Basis des Gehäuses (110) positioniert ist; und
wobei sich Fluid zwischen dem Zylinder (182) und dem Kolben (190) befindet, und
**dadurch gekennzeichnet, dass**
über der länglichen Spitze (188) ein brechbares Siegel (197) angeordnet ist, das so ausgebildet ist, dass es beim Vorschieben des Kolbens (190) in den Zylinder (182) der Pumpvorrichtung (180) bricht.

2. Fluidapplikator (100) nach Anspruch 1, der ferner einen Strömungsverteilungsschwamm (124) aufweist, der benachbart und proximal zur distalen Basis angeordnet ist.

3. Fluidapplikator (100) nach Anspruch 2, wobei der Strömungsverteilungsschwamm (124) so ausgebildet ist, dass er Fluid gleichmäßig von der länglichen Spitze der Pumpvorrichtung (180) verteilt.

4. Fluidapplikator (100) nach Anspruch 1, der ferner eine Reibspitzenkappe (198) aufweist, die über der länglichen Spitze angeordnet ist und so ausgebildet ist, dass sie beim Vorschieben des Kolbens (190) in den Zylinder (182) der Pumpvorrichtung (180) abgeworfen wird.

5. Fluidapplikator (100) nach Anspruch 1, wobei der Zylinder (182) ferner einen Flansch (185) aufweist, der das offene proximale Ende des Zylinders umgibt, wobei der Flansch (185) als harter Anschlag ausgebildet ist, um das Einführen der Pumpvorrichtung (180) in das Gehäuse (110) zu begrenzen.

6. Fluidapplikator (100) nach Anspruch 1, wobei sich eine oder mehrere Längsrippen (116) von einer Innenfläche des Gehäuses (110) erstrecken und so ausgebildet sind, dass sie einen Presssitz mit dem Zylinder erzeugen.

7. Fluidapplikator (100) nach Anspruch 6, wobei die eine oder mehreren Längsrippen (116) Laschen sind.

8. Fluidapplikator (100) nach Anspruch 6, wobei die eine oder mehreren Längsrippen (116) in einem solchen Abstand innerhalb des Gehäuses (110) angeordnet sind, dass das distale Ende des Zylinders (182) der Pumpvorrichtung (180) an die eine oder mehreren Längsrippen anstößt (116).

9. Fluidapplikator (100) nach Anspruch 1, wobei die distale Basis in einem Winkel relativ zum Gehäuse (110) steht.

10. Fluidapplikator (100) nach Anspruch 1, wobei die distale Basis senkrecht zum Gehäuse (110) steht.

11. Fluidapplikator (100) nach Anspruch 1, wobei der Hohlzylinder (182) der Pumpvorrichtung (180) aus einem spritzgegossenen Kunststoff mit hoher Barrierewirkung oder aus Glas besteht, der bzw. das so beschaffen ist, dass er bzw. es gegen Ethylenoxidgas beständig ist.

12. Fluidapplikator (100) nach Anspruch 1, wobei die Pumpvorrichtung (180) beständig gegen Ethylenoxidgas ist.

13. Verfahren zum Zusammenbauen und zum Sterilisieren des Fluidapplikators (100) nach Anspruch 1, das die folgenden Schritte umfasst:
Vorfüllen der Pumpvorrichtung (180) mit Desinfektionsmittel,
Versiegeln der länglichen Spitze der Pumpvorrichtung (180) mit einem brechbaren Siegel (197),
Sterilisieren der Pumpvorrichtung (180) durch Sterilisierung mit Ethylenoxidgas (EtO),
Vorschieben des Hohlzylinders (182) der Pumpvorrichtung (180) in den Hohlraum (114) des Gehäuses (110), und
Verpacken und Sterilisieren des Fluidapplikators (100) mittels einer ungefährlichen Sterilisierung.

## Revendications

1. Applicateur de fluide (100) comprenant :
un boîtier (110) présentant une extrémité proximale ouverte, une base distale et une cavité (114) configurée pour recevoir un dispositif de pompage (180), la base distale présentant une éponge d'applicateur de fluide (122) s'étendant distalement à partir de la base distale ;
le dispositif de pompage (180) étant disposé au moins partiellement à l'intérieur de l'extrémité proximale ouverte du boîtier (110), le dispositif de pompage (180) présentant un cylindre (182) et un piston (190) disposé au moins partiellement à l'intérieur du cylindre, le cylindre (182) présentant une extrémité distale et un embout allongé (188) s'étendant à partir de l'extrémité distale du cylindre, l'embout allongé étant positionné à distance de la base distale du boîtier (110) ; et,
du fluide disposé entre le cylindre (182) et le piston (190), et
**caractérisé en ce que**
un joint frangible (197) est disposé sur l'embout allongé (188) configuré pour se casser lors de l'avancement du piston (190) dans le cylindre (182) du dispositif de pompage (180).

2. Applicateur de fluide (100) selon la revendication 1 comprenant en outre une éponge de distribution de flux (124) disposée adjacente et proximale à la base distale.

3. Applicateur de fluide (100) selon la revendication 2, dans lequel l'éponge de distribution de flux (124) est configurée pour distribuer uniformément le fluide à partir de l'embout allongé du dispositif de pompage (180).

4. Applicateur de fluide (100) selon la revendication 1 comprenant en outre un capuchon d'embout de friction (198) disposé sur l'embout allongé configuré pour être éjecté lors de l'avancement du piston (190) dans le cylindre (182) du dispositif de pompage (180).

5. Applicateur de fluide (100) selon la revendication 1, dans lequel le cylindre (182) comprend en outre une bride (185) entourant l'extrémité proximale ouverte du cylindre, la bride (185) étant configurée comme une butée dure pour limiter l'insertion du dispositif de pompage (180) dans le boîtier (110).

6. Applicateur de fluide (100) selon la revendication 1, dans lequel une ou plusieurs nervures longitudinales (116) s'étendent à partir d'une surface intérieure du boîtier (110) en étant configurées pour créer un ajustement par interférence avec le cylindre.

7. Applicateur de fluide (100) selon la revendication 6, dans lequel les une ou plusieurs nervures longitudinales (116) sont des languettes.

8. Applicateur de fluide (100) selon la revendication 6, dans lequel les une ou plusieurs nervures longitudinales (116) sont positionnées à distance à l'intérieur du boîtier (110) de sorte que l'extrémité distale du cylindre (182) du dispositif de pompage (180) vient en butée contre les une ou plusieurs nervures longitudinales (116).

9. Applicateur de fluide (100) selon la revendication 1, dans lequel la base distale est inclinée par rapport au boîtier (110).

10. Applicateur de fluide (100) selon la revendication 1, dans lequel la base distale est perpendiculaire au boîtier (110).

11. Applicateur de fluide (100) selon la revendication 1, dans lequel le cylindre creux (182) du dispositif de pompage (180) est fabriqué à partir d'un verre ou d'une résine moulée par injection à barrière élevée configuré pour résister au gaz oxyde d'éthylène.

12. Applicateur de fluide (100) selon la revendication 1, dans lequel le dispositif de pompage (180) est résistant au gaz oxyde d'éthylène.

13. Procédé d'assemblage et de stérilisation de l'applicateur de fluide (100) selon la revendication 1 comprenant les étapes de :
préremplissage du dispositif de pompage (180) avec du désinfectant,
scellement de l'embout allongé du dispositif de pompage (180) avec un joint frangible (197), stérilisation du dispositif de pompage (180) par stérilisation au gaz oxyde d'éthylène (EtO),
avancement du cylindre (182) creux du dispositif de pompage (180) dans la cavité (114) du boîtier (110), et,
emballage et stérilisation de l'applicateur de fluide (100) par une stérilisation non dangereuse.
